# EUROPÄISCHE PATENTSCHRIFT

(11) **EP 1 289 522 B1**
(45) Veröffentlichungstag und Bekanntmachung des Hinweises auf die Patenterteilung: **11.05.2005**
(21) Anmeldenummer: 01983259.1
(22) Anmeldetag: 07.06.2001
(51) Int. Cl.: A61K 31/385, A61K 45/06, A61P 25/28

(54) **MITTEL ZUR ADJUVANTEN THERAPIE VON DEMENZEN ENTHALTEND ALPHA-LIPONSÄURE**
MEDICAMENTS FOR THE ADJUVANT THERAPY OF DEMENTIA COMPRISING ALPHA LIPOIC ACID
AGENTS THERAPEUTIQUES ADJUVANT CONTRE LES DEMENCES COMPRENANT DE L'ACIDE LIPOIQUE

(30) Priorität: 08.06.2000 DE 10027968
(43) Veröffentlichungstag der Anmeldung: 12.03.2003
(73) Patentinhaber: VIATRIS GmbH & Co. KG, 61352 Bad Homburg (DE)
(72) Erfinder: WESSEL, Klaus, 61118 Bad Vilbel (DE); MÜNCH, Gerald, 04103 Leipzig (DE); HAGER, Klaus, 30657 Hannover (DE); KENKLIES, Marlene, 30655 Hannover (DE); LOBISCH, Michael, 61203 Reichelsheim/Blofeld (DE); PEUKERT, Manfred, 61440 Oberursel/Ts (DE); BORBE, Harald, 55127 Mainz (DE); MARAHRENS, Andreas, 30916 Isernhagen (DE)
(86) Internationale Anmeldenummer: PCT/EP2001/006478
(87) Internationale Veröffentlichungsnummer: WO 2001/093865

(56) Entgegenhaltungen:
- EP-A- 0 572 922
- WO-A-99/04782
- WO-A-99/06040
- DE-A- 4 309 217
- DE-A- 4 343 592
- US-A- 5 118 505
- US-A- 5 977 162
- STOLL S ET AL: "THE POTENT FREE RADICAL SCAVENGER ALPHA-LIPOIC ACID IMPROVES COGNITION IN RODENTS" ANNALS OF THE NEW YORK ACADEMY OF SCIENCES, NEW YORK ACADEMY OF SCIENCES, NEW YORK, NY, US, Bd. 717, 1994, Seiten 122-128, XP001029748 ISSN: 0077-8923 in der Anmeldung erwähnt
- PACKER L ET AL: "NEUROPROTECTION BY THE METABOLIC ANTIOXIDANT ALPHA-LIPOIC ACID" FREE RADICAL BIOLOGY & MEDICINE, Bd. 22, Nr. 1/2, 1997, Seiten 359-378, XP001038517 in der Anmeldung erwähnt
- DANA CONSORTIUM: "A RANDOMIZED, DOUBLE-BLIND, PLACEBO-CONTROLLED TRIAL OF DEPRENYL AND THIOCTIC ACID IN HUMAN IMMUNODEFICIENCY VIRUS-ASSOCIATED COGNITIVE IMPAIRMENT" NEUROLOGY, Bd. 50, 1998, Seiten 645-651, XP001038541 in der Anmeldung erwähnt

## Beschreibung

Die Erfindung betrifft die Verwendung von α-Liponsäure in reduzierter oder oxidierter Form oder deren Derivaten mit intakter Dithiolanstruktur in Form von Enantiomeren oder pharmazeutisch akzeptablen Salzen, Amiden, Estern, Thioestern, Ethern oder Metaboliten zur Herstellung eines Arzneimittels zur adjuvanten Therapie von Demenzen.

Der Stand der Technik ist niedergelegt in folgenden Veröffentlichungen:
DE 4343592 C2, EP 0382066 A2, DE 4439477 C2, EP 0530446B1, EP 0855396 A1, WO 99/06040, US 5,532,269, DE 4343593 A, US 5,599,835, US 5,569,670, EP 0869126 A1, PCT/GB98/02155, PCT/US99/11178, DE 4327462 A1,
The DANA Consortium. A randomized, double-blind placebo-controlled trial of deprenyl and thioctic acid in human immunodeficiency virus-associated cognitive impairement. Neurology 50: 645-651; 1998,
Di Ricco A, Roesler R, Quevedo J, Walz R, Bianchin M. A randomized, double-blind placebo-controlled trial of deprenyl and thioctic acid in human immunodeficiency virus-associated cognitive impairement. Neurology 52: 1920, 1999,
Boysen KH. Erfahrungen mit dem Präparat Thioctacid auf einer psychiatrischen Station. Med. Welt: 395-400, 1967,
Tirosh O. Sen CK, Roy S, Kobayashi S, Packer L. Neuroprotective effects of α-lipoic acid and ist positively charged amide analogue. Free Rad Biol Med 26 (11/12), 1418-1426, 1999,
Packer L, Tritschler HJ, Wessel K. Neuroprotection by the metabolic antioxidant α-lipoic acid. Free rad Biol Med, 22 (1/2), 359-378, 1997,
Stoll S, Hartmann H, Cohen SA, Müller WE. The potent free radical scavenger α-lipoic acid improves memory in aged mice: putative relationship to NMDA receptor deficits. Pharmacol Biochem Behav 46, 799-805, 1993,
Stoll S, Rostock A, Bartsch R, Kom E, Meichelbock A, Müller WE. The potent free radical scavenger α-lipoic acid improves memory in rodents. Ann NY Acad Sci 717, 122-128, 1994,
Loske C, Neumann A, Cunningham AM, Nichol K, Schinzel R, Riederer P, Münch G. Cytotoxicity of advanced glycation endproducts is mediated by oxidative stress. J Neural Transm, 219-229, 1998,
Hagen TM, Russel T, Ingersoll T, Lykkesfeldt J, Liu J, Wehr CM, Vinarsky V, Batholomew JC, Ames BN. R(+)-alpha-lipoic acid supplemented old rats have improved mitochondrial function, decreased oxidative damage and increased metabolic rate. FASEB J 13: 411-418, 1999 und
Fachinformation Thioctacid, ASTA Medica 1999.

α-Liponsäure (Thioctsäure, 1,2-Dithiolan-3-pentansäure) ist ein biologischer Cofaktor der α-Ketosäuren-Dehydrogenasen in den Mitochondrien und ist an der Biooxidation von diesen Säuren (Pyruvat, α-Ketoglutarat) beteiligt.

α-Liponsäure wird für die Behandlung von Missempfindungen bei diabetischer peripherer Polyneuropathie genutzt sowie zur Behandlung von Erkrankungen der Leber, von Pilz- und Metallvergiftungen. Während früher der therapeutische Effekt oft als unsicher galt, haben neuere Dosis-Wirkungsstudien und die Etablierung einer ausreichend hochdosierten Therapie (> 200 mg pro Tag) in der klinischen Anwendung die Situation gebessert (Fachinformation Thioctacid).

Die biologischen und therapeutischen Effekte der α-Liponsäure in oxidierter und reduzierter Form finden sich auch bei zahlreichen Derivaten und Metaboliten in teils abgeschwächter, teils verbesserter Form (zum Beispiel bei 3-Ketoliponsäure, Liponamid, Octotiamin, 2-(NN-dimethylamine)ethylamidolipoate-HCl, Tocopheryl- und tocotrienyt-Lipoat, gamma-hydroxybuyrat-Lipoat, Liponsäure-Vitamin E-Ester, N-Acetyl-p-Aminophenol-Derivate der Liponsäure und andere) (Tirosch et al, 1999, siehe oben, EP 0855 396 A1 EP 0869126 A1, PCT/GB98/02155, PCT WO 99/06040, DE 4327 462 A1).
Diese Derivate wurden vorgeschlagen, um den Metabolismus und die Verteilung in vivo zu verbessern, was auch für die Verteilung in das zentrale Nervensystem gelten kann. Einzelne Derivate können auch die Effekte (z.B. Affinität und die Umsatzrate) an den biologischen Targets (biologische Redoxsysteme wie α-Ketosäuren-Dehydrogenasen, H-Protein, Thioredoxin, Glutathionreduktase oder zelluläre Redoxsysteme wie Glutathion, Ubichinon, Komplex I der Atmungskette, oder redox- und SH-sensitive Proteine und Enzyme, das NO-System, Katalase, der zelluläre Cystin/Cystein-Shuttle, Homocystein, Tyrosinkinase, MAP-Kinase, Metallionen (zur Komplexbildung), alpha1-Antiproteinase, oder redoxsensitive Transkriptionsfaktoren wie NF-kB oder AP1) von α-Liponsäure verbessern oder andere aktive Moleküle mit dem Ziel synergistischer oder additiver pharmakologischer Wirkung an α-Liponsäure koppeln.

Deshalb wird der Ausdruck "α-Liponsäure" in dieser Schrift als ein genereller Ausdruck verwendet, der außer den Enatiomeren, dem Racemat und Gemischen der Enatiomeren auch Derivate (Ester, Thioester, Ether, Salze, Amide, Metaboliten etc) abdeckt, soweit die aktive Dithiolangruppe der α-Liponsäure für die biologische und medizinische Wirkung des Derivates weiterhin mit verantwortlich ist.

Wegen der Antioxidanzfunktion von α-Liponsäure wurde vorgeschlagen, α-Liponsäure als Nahrungsergänzungsmittel oder Präparat zur medizinischen Ernährung allein oder Kombination mit anderen Stoffen einzusetzen (US 5,569,670, US 5,599,835). Nahrungsergänzungsstoffe oder medizinische Nahrungsmittel sind nach bestimmten nationalen Gesetzen regulierte Produktkategorien, die Herstellung und den Vertrieb von Produkten zu bestimmten Gesundheitszwecken beim Menschen außerhalb des klassischen Arzneimittelbereiches regeln.

α-Liponsäure wurde weiterhin vorgeschlagen zur Behandlung von Zirkulationsstörungen bei Rauchern und Diabetikern (US 5,532,269) sowie als Rheologikum bei Diabetikern (DE 4439477C2).

Es wurde festgestellt, dass das R(+)-Enantiomer vorteilhaft für Bekämpfung entzündlicher Erkrankungen eignet sowie für die Zytoprotektion von Zellen (EP 0382066 A2).

Das R(+)-Enantiomer als ein vorteilhaftes Derivat der racemischen α-Liponsäure wurde für die Behandlung der Insulinresistenz vorgeschlagen (DE 4343593 A1) als auch für die Behandlung von Störungen der Glukoseaufnahme im zentralen Nervensystem (DE 4343592C2).

Die vorteilhafte Verwendung von α-Liponsäure bei Erregungsstörungen des zentralen Nervensystems wurde beschrieben (EP 0530446B1). Wegen der Radikalfängerwirkung wurden umfangreiche Untersuchungen vorgenommen, die die neuroprotektive Wirkung von α-Liponsäure beschreiben. Hierzu gehören Schädigungen des zentralen Nervensystems bei zerebraler Ischämie, Schädigungen durch exzitatorische Aminosäuren, mitochondriale Schäden, kurz gesagt, Hirngewebsschädigungen, bei den freie radikalische Prozesse im Vordergrund stehen (Packer et al., 1997, Loske et al., 1998).

Im Gegensatz zu diesen umfangreichen Untersuchungen zum Schutz der Nervenzellen bei akuten und chronischen Erkrankungen des zentralen Nervensystems liegen bisher nur wenige Daten zur Beeinflussung funktioneller Leistungsverluste des Gehirns vor. Erste Befunde zur Verbesserung dementieller Erscheinungen wurden bei Patienten mit sklerotischer Demenz und bei Involutionsdepressionen nach Gabe von 25-50 mg α-Liponsäure beschrieben (Boysen 1967). Diese Befunde wurden jedoch in der jahrzehntelangen Anwendung von α-Liponsäure nicht bestätigt und müssen wegen der nach heutiger Kenntnis krasser Unterdosierung als fehlführend gelten.

Es wurde dann aber doch beschrieben, dass an alten Ratten α-Liponsäure in hoher Dosis doch zu einer Verbesserung der Gedächtnisleistung alter Ratten führt, nicht jedoch bei jungen Tieren (Stoll et al, 1993, Stoll et al, 1994). Eine positive Beeinflussung motorischer Funktionen bei alternden Tieren wurde für die R(+)-α-Liponsäure beschrieben (Hagen et al., 1999).

Auf Basis dieses Rationals wurde eine erste klinische Studie initiert. Es zeigten sich jedoch keinerlei Effekt von wirksam dosierter (600 mg) α-Liponsäure-Therapie auf die kognitive Funktion von Patienten mit HIV-Demenz, obwohl aufgrund der antioxidativen Eigenschaften der Liponsäure einerseits sowie der zahlreichen tierexperimentellen Befunde zur neuroprotektiven Wirkung sowie zur Verbesserung des Lernverhaltens bei alten Ratten dies erwartet worden war und das Rational der klinischen Studie gebildet hatte. α-Liponsäure war aber nicht etwa ohne Effekt, es kam sogar zu einer Verschlechterung des Lernverhaltens (Di Rocco et al. 1999, Dana Consortium 1998) durch Gabe von α-Liponsäure. Die verbale Lemfähigkeit und das Erinnerungsvermögen nahmen signifikant ab. Alle anderen angewandten neurophysiologischen Tests verschlechterten sich. α-Liponsäure war also nicht wirkungslos, es hatte entgegen der Rationale einen deutlichen pharmakologischen Effekt, der die dementielle Symptomatik verschlechterte. Dieses Ergebnis ist umso enttäuschender, als ein aktives Kontrollarzneimittel (Deprenyl) in dieser Studie seine bekannten positiven Effekte auf die kognitiven Leistungen der dementen Patienten bestätigte und ein negativer Zufallsbefund zu ungunsten von α-Liponsäure deshalb ausgeschlossen werden kann.

Demenz ist eine allgemeine Bezeichnung für die Unfähigkeit des Menschen, die eigenen geistigen Fähigkeiten zu nutzen. Die Demenz als Folge einer Krankheit des Gehirns verläuft gewöhnlich chronisch oder fortschreitend unter Beeinträchtigung vieler höherer kortikaler Funktionen, einschließlich Gedächtnis, Denken, Orientierung, Auffassung, Rechnen, Lernfähigkeit, Sprache und Urteilsvermögen. Die kognitiven Beeinträchtigungen sind meist begleitet von Verschlechterung der emotionalen Kontrolle, des Sozialverhaltens oder der Motivation.
Kriterien für eine Demenz sind u.a. :
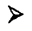 Verlust der intellektuellen Fähigkeiten in solchem Ausmaß, dass soziale und berufliche Fähigkeiten beeinträchtigt sind.
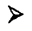 Persönlichkeitsveränderungen einschließlich der Verminderung der Affektkontrolle, des Antriebs oder Vergröberung des Sozialverhaltens (auch emotionale Labilität, Reizbarkeit, Apathie)
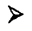 Probleme mit Routinearbeiten
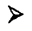 Schwere Gedächtnisprobleme, z.B. Störungen des Kurzzeitgedächtnisses und des Langzeitgedächtnisses Die Fähigkeit, Gemeinsamkeiten und Unterschiede zu erkennen, wird geringer.
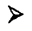 Erschwertes Erlernen neuer Informationen
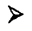 Falsche irrationale Entscheidungen, die zu gefährlichen Situationen führen
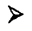 Beginnende Störung des Sprechens und des Verstehens von Sprache (Aphasie)
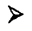 Schwierigkeiten der Bewegungskoordination (Apraxie)
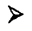 Die Unfähigkeit, Objekte und Menschen zu erkennen (Agnosie)
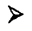 Verlust der Rechenfähigkeit und der Schreibfähigkeit.

Die genauen ursächlichen Krankheitsprozesse sind bis heute nicht endgültig geklärt. Das klinische Bild gleicht sich oft und die pathogenetische Ursache bzw. die Art der Demenz wird oft erst post-mortem feststellbar. Zusätzlich zeigen sich viele Mischformen, bei denen mehrere himorganische Ursachen der Demenz gleichzeitig (oder zeitlich verschoben) nebeneinander vorkommen. Unabhängig von der primären himorganischen Ursache ist für alle Demenzen eine neuronale Dysfunktion ersichtlich. Diese Dysfunktion ist durch eine Störung der Signalübertragung im Gehirn charakterisiert (Neurotransmissionsstörung), wobei die nachlassende Synthese oder verminderte Ausschüttung von verschiedenen Neurotransmittem (wie Acetylcholin, Dopamin und Glutamat, Serotonin und andere) oder verminderte Post-Rezeptorwirkung je nach Krankheitstyp eine individuell verschiedene Rolle spielen. Diese Neurotransmissionsstörung bestimmt das klinische Bild der Demenz bei allen unterschiedlichen himorganischen Ursachen. Im fortgeschrittenen Zustand kann die Dysfunktion bis zum neuronalen Zelltod voranschreiten. Bedeutsam scheinen bei einigen Demenzarten, v.a. der HIV-Demenz und der Alzheimer Demenz, chronisch entzündliche Prozesse zu sein (McArthur et al., 1999; McGeer and McGeer, 1997).
Der klinische Prozess der kognitiven Funktionseinbuße schreitet langsam fort und betrifft meist Menschen im mittleren und älteren Lebensalter. Altem ist der wichtigste Risikofaktor für die Entwicklung einer Demenz (Payami et al., 1997).

Die wichtigsten Demenzarten sind:

### 1. Demenz bei Alzheimer Krankheit

Die Alzheimer-Krankheit ist eine primär degenerative zerebrale Demenz mit unbekannter Ätiologie, aber charakteristischen neuropathologischen und neurochemischen Merkmalen. Sie beginnt gewöhnlich schleichend und entwickelt sich langsam, aber stetig über Jahre (zwei bis drei Jahre, gelegentlich aber mehr). Der Beginn liegt selten im mittleren Erwachsenenalter (Alzheimer-Krankheit mit präsenilem Beginn), häufig jedoch im späteren Lebensalter (Alzheimer-Krankheit mit senilem Beginn). In Fällen vor dem 65. bis 70. Lebensjahr können häufiger familiär ähnliche Fälle beobachtet werden mit rascherem Verlauf und im Vordergrund stehenden Symptomen temporaler und parietaler Schädigung, einschließlich Dysphasie oder Dyspraxie. Fälle mit späterem Beginn neigen zu langsamerem Verlauf und sind durch eine allgemeinere Beeinträchtigung der höheren kortikalen Funktionen charakterisiert. Patienten mit einem Down-Syndrom zeigen ebenfalls eine analoge Form der Alzheimer-Krankheit.

Es finden sich charakteristische Gehimveränderungen: Eine ausgeprägte Verminderung von Neuronen Populationen, besonders im Hippocampus, in der Substantia innominata, dem Locus coeruleus, dem temporo parietalen und frontalen Cortex; Auftreten von neurofibrillären "Tangles" , die aus paarigen, spiraligen Filamenten von Zytoskellettproteinen bestehen; neuritische (argentophile) Plaques, die vorwiegend aus Amyloid bestehen mit einer eindeutig progredienten Entwicklung (aber auch Plaques ohne Amyloid) und granulovakuoläre Körper. Neurochemische Veränderungen sind ebenfalls gefunden worden, so eine deutliche Verminderung des Enzyms Cholin Acetyltransferase, des Acetylcholins und anderer Neurotransmitter und Neuromodulatoren.

Die Verdachtsdiagnose einer Alzheimer-Krankheit wird meist allein aufgrund der klinischen Beurteilung der Symptome gestellt. Neben dem Vorliegen einer Demenz sind schleichender Beginn mit langsamer Verschlechterung charakteristisch. Während der Beginn gewöhnlich nur schwer genau festzustellen ist, kann die Erkenntnis, daß Defizite vorliegen, bei Dritten plötzlich auftreten. Weitere Kriterien sind: Fehlen klinischer Hinweise oder spezieller Untersuchungsbefunde, die auf eine System- oder Himerkrankung hinweisen, welche eine Demenz verursachen kann (z.B. Hypothyreose, Hyperkalzämie, Vitamin-B-12 Mangel, Niazin Mangel, Neurosyphilis, Normaldruck Hydrozephalus, subdurales Hämatom); Fehlen eines plötzlichen apoplektischen Beginns oder neurologischer Herdzeichen wie Hemiparese, Sensibilitätsverlust, Gesichtsfeldausfälle und Koordinationsstörungen in der Frühphase der Krankheit hinzukommen).

### 2. Vaskuläre Demenz:

Die vaskuläre (früher arteriosklerotische) Demenz, einschließlich Multiinfarkt Demenz, unterscheidet sich von der Demenz bei Alzheimer-Krankheit durch den Beginn, die klinischen Merkmale und den Verlauf. Öfter bestehen in der Vorgeschichte transitorisch ischämische Attacken mit kurzen Bewusstseinsstörungen, flüchtigen Paresen oder Visus Verlust. Die Demenz kann auch einer Reihe von akuten zerebrovaskulären Ereignissen folgen oder, weniger häufig, einem einzelnen Schlaganfall. Diese Art der Demenz, die gewöhnlich im höheren Lebensalter beginnt, kann nach einer einzelnen ischämischen Episode abrupt auftreten oder sich allmählich entwickeln. Sie ist gewöhnlich das Resultat einer Infarzierung des Gehirns als Folge einer vaskulären Krankheit, einschließlich der zerebrovaskulären Hypertonie. Die Infarkte sind meist klein, aber kumulieren in ihrer Wirkung.

Die Diagnose setzt eine Demenz voraus. Die kognitive Beeinträchtigung ist gewöhnlich ungleichmäßig, so dass Gedächtnisverlust, intellektuelle Beeinträchtigung und neurologische Herdzeichen auftreten können. Einsicht und Urteilsfähigkeit können relativ gut erhalten sein. Ein plötzlicher Beginn, eine schrittweise Verschlechterung und auch neurologische Herdzeichen und Symptome erhöhen die Wahrscheinlichkeit der Diagnose. Bestätigt werden kann sie in manchen Fällen nur durch Computertomographie oder letztendlich durch die neuropathologische Untersuchung. Als zusätzliche Merkmale kommen vor: Hypertonie, Karötisgeräusche, Affektlabilität mit vorübergehender depressiver Stimmung, Weinen oder unbeherrschtem Lachen und vorübergehende Episoden von Bewußtseinstrübung oder Delir, oft durch weitere Infarkte hervorgerufen. Die Persönlichkeit bleibt meist relativ gut erhalten, aber in einer Anzahl von Fällen können sich Persönlichkeitsänderungen mit Apathie oder Enthemmung oder eine Zuspitzung früherer Persönlichkeitszüge wie Ich-Bezogenheit, paranoide Haltungen oder Reizbarkeit entwickeln.

### 3. Demenz bei Pick-Krankheit

Progrediente Demenz mit Beginn im mittleren Lebensalter gewöhnlich zwischen dem 50. und 60. Lebensjahr, charakterisiert durch frühe, langsam fortschreitende Charakterveränderungen und Verlust sozialer Fähigkeiten. Die Krankheit führt zu Schädigung von Intellekt, Gedächtnis und Sprachfunktionen mit Apathie, Euphorie und gelegentlich auch extrapyramidalen Phänomenen. Das neuropathologische Bild zeigt eine umschriebene Atrophie der Frontal- und Temporal-Lappen, jedoch ohne über das normale Altersmaß hinausgehende neuritische Plaques und neurofibrilläre Verklumpungen. Fälle mit frühem Beginn neigen zu einem malignen Verlauf. Die sozialen- und Verhaltensauffälligkeiten beginnen häufiger vor offensichtlichen Gedächtnisstörungen. Im Gegensatz zur Alzheimer-Krankheit sind Frontalhimsymptome ausgeprägter als Temporal- und Parietalhirnsymptome.

### 4. Demenz bei Parkinson-Krankheit

Eine Demenz, die sich im Verlauf einer bereits bestehenden Parkinson-Krankheit (besonders der schweren Formen) entwickelt. Bisher konnten keine eindeutig kennzeichnenden klinischen Merkmale beschrieben werden. Die Demenz kann von einer Demenz bei Alzheimer-Krankheit oder von vaskulärer Demenz verschieden sein; es gibt jedoch auch Hinweise, dass es sich um ein gleichzeitiges Auftreten einer dieser beiden Demenzformen zusammen mit der Parkinson-Krankheit handelt.

### 5. Lewy-Body-Demenz

Diese Demenzform wird angenommen bei fluktuierender Himleistung (bes. Schwankungen von Aufmerksamkeit und Vigilanz) und zwei von drei der folgenden Punkte:
optische, detailierte Halluzinationen
Parkinsonsymptomatik
Außergewöhnliche Neuroleptika-Sensitivität im Hinblick auf die Entwicklung eines Parkinsonismus

### 6. Demenz bei Krankheit durch das humane Immundefizienz-Virus (HIV)

Eine Störung, die durch kognitive Beeinträchtigungen charakterisiert ist, welche die klinischen Kriterien für die Diagnose einer Demenz erfüllen, bei Fehlen einer gleichzeitig bestehenden Krankheit oder Störung (außer HIV Infektion), die das klinische Bild erklären könnte. Typischerweise bestehen bei der HIV Demenz Klagen wie Vergesslichkeit, Verlangsamung, schlechte Konzentration und Schwierigkeiten beim Lösen von Problemen und beim Lesen. Apathie, verringerte Spontaneität und sozialer Rückzug sind häufig. Bei einer deutlichen Minorität der Betroffenen kann die Krankheit unter dem Bild einer affektiven Störung, einer Psychose oder mit Krampfanfällen auftreten. Bei der körperlichen Untersuchung finden sich häufig Tremor, Störung rascher wiederholter Bewegungen, Gleichgewichtsstörung, Ataxie, Tonussteigerung, allgemeine Hyperreflexie, frontale Enthemmungsphänomene sowie Störungen bei Augenfolgebewegungen und sakkadische Augenbewegungen. Kinder können eine HIV bedingte Entwicklungsstörung des ZNS zeigen, die durch Entwicklungsverzögerung, Tonussteigerung, Mikrozephalie und Verkalkung der Basalganglien charakterisiert ist. Im Unterschied zu Erwachsenen tritt die neurologische Beteiligung meist ohne opportunistische Infektionen und Neoplasmen auf.

Im allgemeinen, aber nicht ausnahmslos, führt die HIV Demenz zu einer schweren, umfassenden Demenz, zu Mutismus und zum Tode.

Neuropsychiologische Messverfahren zum Schweregrad der Demenz Für ein Demenzscreening im klinischen Alltag werden standardisierte Verfahren verwendet, z.B. die Mini-Mental-State-Untersuchung (MMSE) oder der Alzheimer's Disease Assessment Scale (ADAS). Weitere Verfahren wären: GDS: Global Deterioriation Scale (Reisberg et al., 1982), FAST: Functional Assessment Staging (Reisberg, 1988).

### Mini Mental State Examination (MMSE)

Der Mini-Mental State Examination Test wurde von Marshall Folstein 1975 veröffentlicht, war ursprünglich als Test zur Unterscheidung der kognitiven Funktionen gedacht (Folstein et al., 1975). Auf Grund der Kürze des Tests wird er heute als Screening Test bei Studien zur Erfassung von Ein / Ausschlußkriterien verwandt. Getestet werden Orientierung, Aufmerksamkeit, kurz- und mittelfristige Merkfähigkeit, Wortfindung, Lesen, Schreiben, weiterhin muss eine Handlungsanweisung befolgt werden. Die Aufgaben des Tests sind so konzipiert, dass sie von kognitiv nicht beeinträchtigten Personen normalerweise problemlos gelöst werden können. Die höchste erreichbare Punktzahl beträgt 30 Punkte, weniger als 24 Punkte weisen auf eine dementielle Erkrankung hin. Folgende Stadien werden im allgemeinen unterschieden (Zec et al., 1992).

| | | |
|---|---|---|
| MMSE | mehr als 24 Punkte | = sehr wenig auffällig, |
| | 20 -23 Punkte | = auffällig, |
| | 10 - 19 Punkte | = sehr auffällig, |
| | 0 - 9 Punkte | = schwere |

### Alzheimer's Disease Assessment Scale-Cognitive Subscale (ADAS-Cog)

Ein weiterer Test zur Ermittlung des neuropsychologischen Status eines Demenzpatienten ist der "Alzheimer's Disease Assessment Scale-Cognitive Subscale (ADAS-Cog)" (Rosen et al., 1984). Die Alzheimer Disease Assessment Scale wurde von W.G.Rosen, R.C. Mohs, 1988, Rosen u. K.L. Davis, 1984 erfunden, die deutsche Bearbeitung erfolgte von R.lhl und G. Weyer, Beltz Test 1993. Der ADAS war ursprünglich nicht als diagnostisches Instrument gedacht, trennt aber sehr gut demente Personen von gesunden altersgleichen Kontrollpersonen. Im ADAS können folgende Kriterien unterschieden werden: kognitiver Bereich, nichtkogn. Bereich, Gedächtnis, Orientierung/ Praxie, Motorik, Depressivität, Psychotische Symptome, Konzentration/Kooperation, Sprache. In der hier benutzten Fassung wird die Sprache in den kognitiven Bereich mit hineingenommen, es können so max. 70 Minuspunkte gemacht werden. Für Personen mit einer Demenz vom Alzheimer Typ konnte gezeigt werden, dass die Gesamtskala im Laufe von 12 Monaten im Mittel um 5 bis 7 Punkte ansteigt, im Laufe von 24 Monaten um durchschnittlich 14 Punkte. Hier sind die ltems des kognitiven Teils am sensitivsten.
Wie beim MMSE kann man auch bei der ADAS eine Einteilung vornehmen:

| | | |
|---|---|---|
| ADAS | weniger als 23 Punkte | = sehr wenig auffällig, |
| | 24 - 38 Punkte | = auffällig, |
| | 39 - 53 Punkte | = sehr auffällig, |
| | mehr als 54 Punkte | = schwer. |

### Die Therapie der Demenzen

Alle heute verfügbaren Wirkstoffe im Bereich Demenzen (einschließlich der Nootropika) bringen leider in der Therapie nur marginale Verbesserungen für den Patienten bzgl. der symptomatischen Himleistungsdefizite.
Die Medikamente bringen oft nur für wenige Monate Verbesserung und müssen anhand des Verträglichkeitsprofils sorgsam ausgewählt werden. Schon eine Verlangsamung des Fortschreitens des kognitiven Funktionsverlustes muss als therapeutischer Fortschritt gesehen werden. Seiten nur sprechen Patienten so gut an, daß es sogar zu einer vorübergehenden Verbesserung der Hirnleistung kommt, die aber nicht zu einer dauerhaften Abstoppung des Krankheitsverlaufs führt.

Allen Wirkstoffen ist gemeinsam, dass auf unterschiedlichste Art und Weise die gestörte neuronale Signaltransmission wieder verbessert wird. Das gesagte sowohl für die Wirkstoffe, die die Himdurchblutung verbessern (Ginkgo-Extrakte, Cinnarizin, Hihydroergotoxine, Nicergolin, Piracetam, Pentoxyfillin, Pyritinol, Vinpocetin), die Leistungsfähigkeit der Nervenzellen verbessern oder Nervenzellen schützen (Calciumantagonisten, NMDA-Antagonisten, Memantine) oder auf andere Art die Signalübertragung im Gehirn verbessern (z.B. Pro-Acetylcholinergika wie z.B Aricept (Wirkstoff: Donezepil), Exolon (Wirkstoff: ENA 713), oder Rivastigmin).

Aufgrund des immensen Bedarfs an weiteren wirksamen, gut verträghlichen Arzneimitteln gibt es eine Reihe von Stoffen in der klinischen Entwicklung (Galantamin, Propentofyllin).Weiterhin werden auch Entzündungshemmer ("nicht steroidale Antiphlogistika" wie lbuprofen, Acetylsalicylsäure oder Diclofenac) eingesetzt. Weiterhin wird noch untersucht, ob Stoffe wie Vitamin E einen klinischen Effekt zeigen.

Zusammenfassend lässt sich sagen, dass es bis heute leider erst sehr wenige therapeutische Ansätze gibt, welche bestenfalls einen vorübergehend stabilisierenden Einfluss haben, nicht aber einen langanhaltenden Schutz vor der weiteren Progression der Demenz bieten.

Nun wurde überraschend gefunden, dass die adjuvante Gabe von α-Liponsäure in ausreichender Dosis zu einer Basistherapie mit Pro-Acetylcholinergika in besonderer Weise die Leistungsdefizite dementer Personen verbessert bzw. die natürliche Verschlechterung der Ausfallerscheinungen aufhält oder stoppt.

Dieses Verhalten eines Arzneistoffes, nur in der Kombination eine klare und ausgeprägte positive Wirkung zu zeigen, ist ungewöhnlich. Üblicherweise kann es in der Kombination zweier als Monotherapie wirksamer Stoffe zu einem additiven oder synergistischen positiven Effekt kommen. Eine fehlende gar oder negative Wirkung eines Wirkstoffes in der Monotherapie, die sich aber dann in der Kombination als positiv manifestiert bzw. ins Positive umkehrt, ist unerwartet.

### Beispiele

Patienten mit klinisch manifesten dementiellen Ausfallerscheinungen und Verdacht auf Hirnschäden auf Basis eines Alzheimer Demenz oder einer vaskulär bedingten Demenz oder ähnlicher Verdachtsdiagnose wurden mit Medikamenten zur Verbesserung der symptomatischen Himleistungsdefizite (Nootropika) behandelt. Da alle heute verfügbaren Wirkstoffe im Bereich Demenzen nur marginale Verbesserungen mit sich bringen, war trotz der enttäuschenden ersten Studie gerechtfertigt, auf Basis dieser Basistherapie Patienten in hoher Dosis α-Liponsäure verabreicht. Die Wirkung der Therapie wurde mit heute üblichen standardisierten klinischen Bewertungsskalen der Demenz beurteilt, die sich durch eine hohe Zuverlässigkeit auszeichnen. Es handelt sich um die Mini-Mental-State-Untersuchung (MMSE) oder der Alzheimer's Disease Assessment Scale (ADAS). Der Vorteil dieser klinischen Instrumente ist, dass der Schweregrad der Demenz zuverlässig erfasst werden kann, unabhängig von der in der Regel nicht eindeutigen himorganischen Basis des klinischen Demenzbildes.

### Patienten mit Alzheimer Demenz

### Beispiel 1:

Patient 1 (geb. 1945), m., Diagnose einer Alzheimer-Demenz 12/ 98, bei Beginn der Liponsäure-Therapie: GDS: 3/4, mäßige kognitive Einbußen, FAST: verminderte Fähigkeiten, komplexe Aufgaben nachzuvollziehen, verliert seine Arbeit, da alleinstehend entsteht eine große Unsicherheit bei dem Pat., braucht Hilfen im Umgang mit Ämtern und bei der Planung seiner tägl. Arbeiten im Haus.
Der Patient bekommt seit 4 Monaten Aricept (5/10 mg/ Tag), wobei sich der kognitive Zustand sich um 1 Punkt im MMSE im MMSE verschlechterte. Nach 4 Wochen Zusatztherapie mit 600mg α-Liponsäure verbesserte sich der MMSE um 2 Punkte, der ADAS nahm um 6 Punkte auf 18 Punkte ab. Pat. kann seinen Namen und seine Adresse wieder leserlich schreiben, kann Fahrrad fahren und sich sportlich betätigen.

### Beispiel 2:

Patient 2 (geb. 1948) w, Diagnose einer Alzheimer-Demenz 10/97, bei Beginn der Liponsäure-Therapie: GDS: 5/6, mäßig schwere Einbußen, FAST: braucht viel Hilfe bei der Verrichtung der tägl. Dinge, lebt bei den Eltern, die für die Tochter sorgen müssen.
Die Patientin bekommt seit 12 Mon. Aricept (5/10mg/Tag). Der kognitive Stand hat sich kontinuierlich verschlechtert; im MMSE um 7 Punkte, im ADAS auf 53 Punkte.
Auf Grund einer demenztypischen depressiven Verstimmung nimmt die Pat seit 5 Mon. Remotiv (Johanniskraut) und seit 12 Mon. 600mg Vitamin E. Mit Aufnahme der Zusatztherapie von 600mg Liponsäure verbesserte sich die Kompliance der Pat. erheblich, im MMSE gab es eine Punktverbesserung von 1 Punkt im Verlauf der nächsten 7 Monate, im ADAS um 11 Punkte.

### Beispiel 3:

Patient 3 (geb.1930) m, Diagnose einer Alzheimer-Demenz 5/97, bei Beginn der Liponsäure-Therapie: GDS: 4/5, mäßige kognitive Einbußen, FAST: braucht viel Hilfe durch seine Ehefrau.
Der Pat. wird seit 13 Mon. mit Aricept (5mg/Tag) behandelt, zusätzlich mit 600mg Vitamin E. Der MMSE verschlechterte sich in dieser Zeit um 2 Punkte, der ADAS nahm um 14 Punkte zu. Nach Zusatztherapie mit 600mg Liponsäure verbesserten sich die kognitiven Funktionen im MMSE um 1 Punkt, im ADAS wurden 2 Punkte wieder aufgeholt. Der Pat. fühlt sich wohler und kann seine Frau wieder öfter begleiten. Er kann kleine Einkäufe erledigen, da sich das Geschäft in unmittelbarer Nähe seiner Wohnung befindet. Der GDS verbesserte sich auf 4.

### Beispiel 4:

Patient 4 (geb. 1940) m, Diagnose einer Alzheimer-Demenz 11/96, bei Beginn der Liponsäure-Therapie: GDS: 3/4 mäßige kognitive Einbußen, Verlust der Berufstätigkeit, FAST: braucht Hilfe durch die Ehefrau.
Behandlung mit Aricept seit 19 Mon.(5/10 mg/Tag), seit 20 Mon. mit 300mg ASS und 600mg Vitamin E. Die kognitive Leistungsfähigkeit verringerte sich um 5 Punkte im MMSE und 9 Punkte im ADAS. Nach Einsatz von 600mg Liponsäure seit 8 Mon. Verbesserung im MMSE um 2 Punkte und im ADAS ging der Wert um 3 Punkte zurück. Der Pat. kann sich wieder an Gesprächen beteiligen, Sport betreiben und kleine Aufgaben im Haushalt durchführen.

### Beispiel 5:

Patient 5 (geb. 1931) w, Diagnose einer Alzheimer-Demenz 5/1998, bei Beginn der Liponsäure-Therapie: GDS: 4/5 mäßige kognitive Einbußen, FAST: braucht Hilfe bei allen Arbeiten im Haus, Wäsche, Kleidung.
Behandlung mit Aricept (5/10mg/Tag) seit 24 Mon., weitere Behandlung mit Accuzide 20 (Blutdruck senkendes Mittel), mit Akatinol und 600mg Vitamin E. Die Pat. verschlechterte sich trotz dieser Therapie um 6 Punkte im MMSE. In den ersten 3 Mon. nach Gabe von 600mg Liponsäure verbesserte sich die Pat. um einen Punkt im MMSE und um eine Stufe in der GDS, jetzt Stufe 4. Der Zustand hält z.Z. an. Die Pat. zeigt ebenso Verbesserung in der koordinierten Motorik, so kann sie wieder in Begleitung Rad fahren und einige Dinge im Garten erledigen.

### Beispiel 6:

Patient 6 (geb. 1919) m, Diagnose einer Alzheimer-Demenz 9/1996, bei Beginn der Liponsäure-Therapie: GDS: 4/5 mäßige kognitive Einbußen, FAST: braucht Hilfe in allen lebenspraktischen Bereichen.
Der Pat. wird seit 13 Mon. mit Aricept (5/10mg/Tag) behandelt, seit 5 Jahren nimmt er ASS 100, seit 3 Jahren Rökan (Gingko biloba) und 600mg Vitamin E. Trotzdem zeigte sich eine Verschlechterung der Demenz um 5 Punkte im MMSE. Seit Behandlung mit 600mg Liponsäure kommt es zu keiner weiteren Verschlechterung, der ADAS hat um 8 Punkte abgenommen. Der Pat. kann an vielen Aktivitäten außer Haus teilnehmen, der GDS verbesserte sich auf 4.

### Beispiel 7:

Patient 7 (geb. 1939) m, Diagnose einer Alzheimer-Demenz 4/97, bei Beginn der Liponsäure-Therapie: GDS: 4 kogn. Einbußen, so dass der Pat. seine leitende Stellung aufgeben musste, FAST: Probleme im Umgang mit Behörden und komplexen Sachvorgängen.
Der Pat. wird seit 6 Mon. mit Aricept (5mg/Tag) behandelt, zusätzlich mit 600mg Vitamin E. Die Demenz verschlechterte sich in dieser Zeit um 6 Punkte im MMSE. Nach Gabe von 600mg Liponsäure kam es bald zu einem Stopp der Progression und nach zwei Mon. zu einer leichten Verbesserung um einen Punkt im MMSE, der ADAS bleibt konstant. Der Pat. kann sich mehrere Tage mit wenig Hilfe allein versorgen, da die Ehefrau verreisen musste. Er kann wieder kleine Einkäufe tätigen.
Bei UB zeigte sich, dass die Therapie mit α-Liponsäure auch dann erfolgreich fortgeführt werden kann, wenn die Basistherapie versuchsweise wieder entzogen wird.

### Beispiel 8:

Patient 8 (geb.1942) m, Diagnose einer Alzheimer-Demenz 6/99, bei Beginn der Liponsäure-Therapie: GDS: 3, Pat. muß seinen Beruf aufgeben, da er die Fähigkeit des Rechnens verloren hatte.
Behandlung mit Aricept (5/10mg /Tag) seit 8 Wochen, seit 4 Wochen Behandlung mit 600mg Liponsäure. Der MMSE liegt zu Beginn der Behandlung mit 600 mg Liponsäure bei 18 Punkten, jetzt 20 Punkte; der ADAS ist um 3 Punkte auf 35 Fehlerpunkte zurückgegangen.

### Patienten mit anderen Demenzarten

### Beispiel 9:

Patient 9 (geb. 1938) m, Diagnose einer Lewy-Körper Demenz 7/1996; GDS: 5/6 schwere kognitive Beeinträchtigungen, FAST: braucht bei allen Verrichtungen des täglichen Lebens Hilfe durch die Ehefrau.
Der Pat. wird 12 Mon. mit Aricept 5mg behandelt, Madopar T 1X125/ täglich und Madopar Depot für die Nacht. Im MMSE erreichte der Pat. 17 Punkte. Nach Behandlung mit 600mg Liponsäure kommt es zu einer Verbesserung im MMSE um 3 Punkte, die Sprache des Pat. verbessert sich, sodaß er wichtige Dinge wieder seiner Frau vermitteln kann, es kommt zu einem Stop der Progression. Der Pat. kann an vielen Alltagsaktivitäten besser teilnehmen.

### Beispiel 10:

Patient 10 (geb. 1929) m, Diagnose einer Frontalhirnatrophie 3/1999, GDS: 3, FAST: Pat. ist leicht aggressiv und dadurch von der Ehefrau sehr schwer zu führen.
Der Pat. wird seit 9 Monaten mit Exelon 6mg/Tag behandelt, der MMSE nahm in dieser Zeit um 5 Punkte ab. Nach zusätzlicher Behandlung mit 600mg Liponsäure konnte eine Verbesserung im MMSE um 1 Punkt erreicht werden. Der Pat. kann wieder allein spazieren gehen und sich an vielen Alltagaktivitäten beteiligen, z.B. Rad fahren.

### Beispiel 11:

Patient 11 (geb. 1925) m, Diagnose einer Demenz plus Parkinson 11/96; GDS: 5, FAST : Pat. braucht bei allen täglichen Verrichtungen Hilfe durch die Ehefrau.
Pat. wird seit 17 Mon. mit Aricept 5/10mg/Tag behandelt, seit 6/1999 zusätzlich mit Madopar T (3x125 mg) und seit 4 Mon. zusätzlich mit 600mg Liponsäure. Die bis dahin beobachtete Progredienz konnte gestoppt werden, eine Verschlechterung ist z. Z. nicht erkennbar. Die Werte im MMSE und ADAS sind auf dem gleichen Stand geblieben. Der Pat. kann an kleinen Alltagsaktivitäten wieder besser teilnehmen.

### Beispiel 12:

Patient 12 (geb. 1935) w, Diagnose einer Frontalhimatrophie seit 4/1966; GDS: 3, FAST: Pat. fällt durch Aggressivität auf, hinzu kommen kog. Defizite und eine motorische Aphasie.
Sie wird mit Zoloft 50 (Psychopharmaka), ASS100, Gingko biloba, Eunerpan (Psychopharmaka , abgesetzt) behandelt und seit 8 Wochen mit Aricept (5/10 mg/Tag) und seit 4 Wochen mit 600mg Liponsäure. Der MMSE geht in dieser kurzen Zeit von 16 auf 18 Punkte zurück und der ADAS veringert seine Punktzahl um 5 von 41 Punkte auf 36 Punkte. Die Kompliance der Pat. nimmt deutlich zu (aggressive Phasen), so dass der Ehemann mit der Betreuung seiner Frau z.Z. wesentlich besser zurecht kommt

### Pharmazeutische Beispiele

α-Liponsäure oder Derivate können in oralen Dareichungsformen (Tabletten oder Kapseln mit schneller oder retardierter Freisetzung, Trinklösungen oder Suspensionen) oder parenteral intravenös oder intramuskulär aus Ampullen oder Fertiginfusionen nach dem generellen Stand der Technik hergestellt werden, wie er zum Beispiel in den unten aufgeführten Dokumenten beschreiben ist.

EP 901211340.5, EP 0858 802 A2, EP 0318 891 A1, EP 0 560 092 B1, US 005650429A, US 005334612A, US 005569670A, US 9755433 A, PCT/US99/11178 und Japanische Offenlegungsschrift (hei) 1 _ 335772 (25.12.1989).

Die so hergestellten Produkte können für den Zweck des Einsatzes bei Demenzen beschriftet in den Verkehr gebracht werden, wobei für die Anleitung der Anwendung für Fachpersonal und Patient die entsprechenden nationalen Regeln zu beachten sind. Die Produkte können sich hierbei üblicherweise im Rechtsrahmen von Arzneimitteln oder gegebenenfalls auch von Nahrungsergänzungsstoffen bewegen.

Es können wirksame Dosierungen von α-Liponsäure als Adjuvanz einer Demenztherapie verwendet werden. Die Demenzen können auf unterschiedlichster hirnorganischer Grundlage (Alzheimer, Pick-Demenz, vaskuläre Demenz, Parkinsondemenz u.a.m. oder auch auf Basis der häufigen Mischformen) vorliegen.

Hierbei besteht die Basistherapie in der Verabreichung von Pro-Acetylcholinergika. Die α-Liponsäure kann als Zusatztherapie bei leichter, mittlerer oder schwerer Demenz zu einem oder mehreren Demenzprodukten gegeben werden oder nach einer erfolgten Vorbehandlung bis zum Auftreten einer bedeutsamen Verschlechterung auch zeitweise allein gegeben werden.

Für eine Therapie kommen auch prädementielle Patienten mit distinkten chronischen oder passagären Einschränkungen der kognitiven Fähigkeiten oder der Aktivitäten des täglichen Lebens oder allgemeiner Tonuseinschränkung in Frage. Diese Patientengruppe wird in der Literatur als MCI-Patienten bezeichnet (mildly congnitively impaired). Im Vordergrund_bei prädementiellen Patienten bzw. klinisch noch wenig auffälligen Patienten mit leichter Demenz stehen präventive Aspekte, wie eine möglichst lange Erhaltung der beruflichen und privaten Leistungsfähigkeit und Berufsfähigkeit und eine möglichst lange Vermeidung von Pflegebedürftigkeit, ohne das diese therapeutischen Ziele nicht auch für voll demente Patienten eine Bedeutung hätten.

Im Verlauf der α-Liponsäure-Gabe können auch weitere Produkte zur Therapie von Hirnleistungsstörungen gegeben werden (Polytherapie) oder psychiatrische Maßnahmen in das individuelle Behandlungskonzept integriert werden.
Weitere Ziele der Therapie können neben der eigentlichen Verbesserung kognitiver Funktionen sein: Verbesserung der gestörten motorischen Leistungsfähigkeit und des körperlichen und mentalen Tonus, Milderung der depressiven und psychiatrisch-psychotischen Begleitsymptomatik, Verbesserung der Compliance des Patienten für die eigentliche Basistherapie sowie für das nicht-medikamentöse individuelle Therapieregime, auch Verbesserung der Verträglichkeit des gesamten therapeutischen Regimes, Herabsetzung der Pflegebedürftigkeit und des Pflegeaufwands, Verbesserung der Aktivitäten des täglichen Lebens, gegebenenfalls Wiederherstellung der Berufsfähigkeit. Letztere Punkte können auch im Rahmen eines prädementiellen Therapiekonzeptes von Wichtigkeit sein.

α-Liponsäure kann in oraler oder parenteraler Verabreichung (i.v., i.m.) gegeben werden, wobei für die orale Therapie feste oder flüssige schnell freisetzende oder retadiert freisetzende pharmazeutische Formulierungen zum Einsatz kommen können.

Es können Dosierungen von 100-2000 mg α-Liponsäure oder äquivalente molare Mengen eines Derivates - wobei als molare Einheit die intakte Dithiolanstruktur zu sehen ist - als Tagesdosis gegeben werden, wobei die Tagesdosis als Einmalgabe oder in geteilten Dosen über den Tag verteilt gegeben werden kann, vorzugsweise 1-3 Dosierungen. Vorzugsweise werden 300-1200 mg α-Liponsäure als Tagesdosis gegeben wird, wobei die Tagesdosis als Einmalgabe oder in 2-3 geteilten äquivalenten Dosen über den Tag verteilt gegeben werden kann.

Derivate der α-Liponsäure in reduzierter oder oxidierter Form (z.B. Salze, Ester, Thioester, Ether, Amide, Metabolite) analog eingesetzt werden können, soweit sie in einer Dosis verabreicht werden, dass äquivalente Konzentrationen oder biologische Effekte an den Zielstrukturen (biologische Redoxsysteme) erreicht werden

Vorzugsweise wird optisch rechtsdrehende α-Liponsäure (R(+)-α-Liponsäure oder R(-)-Dihydroliponsäure) eingesetzt. α-Liponsäure oder deren Derivate können in freier oder fixer Kombination mit einem oder mehreren Wirkstoffen zur Verbesserung von Himleistungsstörungen in wirksamer Kombination verabreicht wird.

Es können Vitamin C und Derivate, Vitamin E und Tocopherole, Ubichinon, Vitamin B1-12, Thiamin, Riboflavin, Pantothensäure, Niacin, Biotin, Inositol, β-Carotin, Zink, Magnesium, Selen, Taurine, Cholin, N-Acetylcystein und Derivate, ungesättigte Fettsäuren, essentielle Fettsäuren der n-3 und n-6-Reihe, gamma-Linolensäure und Derivate, Aspirin und nichtsteroidale Antiphlogistika, L-Carnitin und Derivate, Johanniskraut, Knoblauchpräparationen in freier oder fester Kombination der α-Liponsäure zugesetzt werden können.

α-Liponsäure und die Basiswirkstoffe zur Therapie von Himleistungsstörungen können in Form der Produktkategorien Arzneimittel, Nahrungsergänzungsstoffe oder medizinischen Emährungsmitteln verarbeitet und angewandt werden.

## Patentansprüche

1. Verwendung von alpha-Liponsäure in reduzierter oder oxidierter Form oder deren Derivaten mit intakter Dithiolanstruktur in Form von Enantiomeren oder pharmazeutisch akzeptablen Salzen, Amiden, Estern, Thioestern, Ethern oder Metaboliten zur Herstellung eines Arzneimittels zur adjuvanten Therapie von Demenzen mit einer Basistherapie von Pro-Acetylcholinergika.

2. Verwendung nach Anspruch 1 von R-(+)-alpha-Liponsäure in oxidierter oder reduzierter Form als Derivat von alpha-Liponsäure.

3. Verwendung nach Anspruch 1 oder 2, wobei zusätzlich ein oder mehrere Demenztherapeutika oder ein oder mehrere Wirkstoffe zur Verbesserung der Neurotransmission verwendet werden.

4. Verwendung nach einem der Ansprüche 1 bis 3, wobei zusätzlich ein oder mehrere der folgenden Stoffe ausgewählt aus Vitamin C und Derivaten, Vitamin E und Tocopherolen, Ubichinon, Vitamin B1-12, Thiamin, Riboflavin, Pantothensäure, Niacin, Biotin, Inositol, beta-Carotin, Zink, Magnesium, Selen, Taurine, Cholin, N-Acetylcystein und Derivaten, ungesättigten Fettsäuren, essentiellen Fettsäuren der n-3 und n-6-Reihe, gamma-Linolensäure und Derivaten, Aspirin und nichtsteroidalen Antiphlogistika, L-Camitin und Derivaten, Johanniskraut und Knoblauchpräparationen, verwendet wird.

5. Verwendung nach einem der Ansprüche 1 bis 4 bei prädementen Patienten.

6. Verwendung nach einem der Ansprüche 1 bis 4, wobei alpha-Liponsäure als adjuvante Therapie zu dem Pro-Acetylcholinergikum nach einer erfolgten Vorbehandlung bis zum Auftreten einer bedeutenden Verschlechterung zeitweise allein gegeben wird.

## Claims

1. Use of alpha-lipoic acid in reduced or oxidized form or derivatives thereof with intact dithiolane structure in the form of enantiomers or pharmaceutically acceptable salts, amides, esters, thioesters, ethers or metabolites for producing a medicament for the adjuvant therapy of dementias with a basic therapy of pro-acetylcholinergic agents.

2. Use as claimed in claim 1 of R(+)-alpha-lipoic acid in oxidized or reduced form as derivative of alpha-lipoic acid.

3. Use as claimed in claim 1 or 2, where there is additional use of one or more dementia therapeutic agents or one or more active ingredients for improving neurotransmission.

4. Use as claimed in any one of claims 1 to 3, where there is additional use of one or more of the following substances, selected from vitamin C and derivatives, vitamin E and tocopherols, ubiquinone, vitamin B1-12, thiamine, riboflavin, pantothenic acid, niacin, biotin, inositol, beta-carotene, zinc, magnesium, selenium, taurines, choline, N-acetylcysteine and derivatives, unsaturated fatty acids, essential fatty acids of the n-3 and n-6 series, gamma-linolenic acid and derivatives, aspirin and non-steroidal antiinflammatory drugs, L-carnitine and derivatives, St. John's wort and garlic preparations.

5. Use as claimed in any one of claims 1 to 4 for predementia patients.

6. Use as claimed in any one of claims 1 to 4, where alpha-lipoic acid is given alone at times as adjuvant therapy to the pro-acetylcholinergic agent after pretreatment has taken place until a significant deterioration appears.

## Revendications

1. Utilisation d'acide alpha-lipoïque sous forme réduite ou oxydée ou de ses dérivés, ayant une structure de dithiolane intacte, sous la forme d'énantiomères ou de sels, d'amides, d'esters, de thioesters, d'éthers ou de métabolites, acceptables du point de vue pharmaceutique, en vue de la fabrication d'un médicament pour la thérapie adjuvante des démences avec une thérapie de base de pro-acétylcholinergiques.

2. Utilisation, selon la revendication 1, d'acide R-(+)-alpha-lipoïque sous forme oxydée ou réduite en tant que dérivé de l'acide alpha-lipoïque.

3. Utilisation selon la revendication 1 ou 2, un ou plusieurs produits thérapeutiques de démence ou un ou plusieurs agents actifs en vue de l'amélioration de la neurotransmission étant utilisés en supplémentaire.

4. Utilisation selon l'une quelconque des revendications 1 à 3, une ou plusieurs des substances, sélectionnées parmi la vitamine C et ses dérivés, la vitamine E et des tocophérols, l'ubiquinone, la vitamine B1-12, la thiamine, la riboflavine, l'acide pantothénique, la niacine, la biotine, l'inositol, la β-carotine, le zinc, le magnésium, le sélénium, la taurine, la choline, la N-acétylcystéine et dérivés, des acides gras insaturés, des acides gras essentiels de la série n-3 et n-6, l'acide gamma-linolénique et dérivés, l'aspirine et des antiphlogistiques non stéroïdiques, la L-carnitine et dérivés, l'herbe de Saint-Jean, des préparations d'ail, étant utilisées en supplémentaire.

5. Utilisation selon l'une quelconque des revendications 1 à 4 dans le cas de patients pré-démentiels.

6. Utilisation selon l'une quelconque des revendications 1 à 4, l'acide alpha-lipoïque étant pris, à lui seul, par moments, en tant que thérapie adjuvante au pro-acétylcholinergique après un traitement préalable réussi jusqu'à l'apparition d'une aggravation significative.
